# EUROPEAN PATENT APPLICATION

(11) **EP 0 600 557 A2**
(43) Date of publication of application: **08.06.1994**
(21) Application number: 93203337.6
(22) Date of filing: 29.11.1993
(51) Int. Cl.: A61F 2/42

(54) **Prosthesis for replacing middle interphalangeal joint**

(30) Priority: 28.11.1992 DE 9216202 U
(71) Applicant: ESKA Implants GmbH & Co., 23556 Lübeck (DE)
(72) Inventor: Schug, Martin, D-23552 Lübeck (DE); Rischke, Burkhard, D-25462 Rellingen (DE)
(74) Representative: Fuchs, Luderschmidt & Partner Patentanwälte

(57) **Abstract**

Prosthesis for replacing a middle interphalangeal joint consisting of two parts joined together by means of a hinge, where each part has a shaft for anchoring in the adjacent finger bone, characterised in that the first part (1) and the second part (3) are joined together by means of a hinge (30). The advantage of this design is that it is simple and easily assembled during surgery.

## Description

This invention concerns a prosthesis for replacing a middle interphalangeal joint, consisting of two parts that are joined by means of a hinge, whereby each part has a shaft for anchoring in the adjacent finger bone.

A finger joint endoprosthesis consisting of two parts that are hinge-connected to each other each having a shaft on the free ends opposite each other for insertion into a finger bone is known from British Patent 1,582,974. The parts are designed in such a way that they are interlocking in order to assure their functionality, which thus results in a very complicated design of the parts and can result in problems in joining the parts during surgery.

This invention is therefore based on the object of creating a middle interphalangeal joint prosthesis of the above-mentioned type that will have a simpler design and can be joined together more easily during implantation.

This object is achieved with the middle interphalangeal joint prosthesis of the type described above due to the fact that the two parts that are to be joined together with an articulation are connected by means of a hinge.

The hinge offers the advantage that it permits a relatively simple and easy means of creating an articulated connection of the parts that are to be first anchored individually in the adjacent bone and then hinge-connected to each other during implantation under the relatively difficult conditions prevailing during surgery.

In an advantageous embodiment of this invention, one part of the prosthesis is designed as a fork with two projections having some distance between them and provided with aligned boreholes, and the second part of the prosthesis is designed with a projection that also has a borehole and can be inserted into the interspace between the two projections on the first part, in which case an axle can be inserted through the aligned boreholes in order to form the hinge. According to another advantageous embodiment, the projections on the first part run parallel to each other.

The fork-shaped design of one part of the hinged prosthesis permits a simple method of inserting the counterpart into the interspace between the fork projections, so that when the boreholes in the two parts are aligned, one need only insert the axle through the boreholes in order to make the joint functional. Despite the simple design, the endoprosthesis satisfactorily meets the movement requirements to be made of a finger joint prosthesis, especially with regard to flexion and extension of the finger.

Additional advantageous embodiments of this invention are described below.

Thus, in a preferred embodiment, a conical snap connector may also be used instead of the cylindrical axle passing through the boreholes. The axle may also be designed to be conical over its entire length or just a portion of the length. To accomplish this, the projections must be provided with appropriate boreholes of different diameters or with conical bores. The conical bore may be provided in one of the projections on the first part, and then the bores in the other two projections will have the same (larger) diameter. In an alternative embodiment, the conical bore is provided in the projection on the second part, so the bore in one projection on the first part must have the same diameter as the end of the cone, and the bore in the other projection on the first part must have the same diameter as the beginning of the cone. If an axle that is conical over the entire length is used, all the bores must be designed to be conical accordingly.

With the conical snap connector, the two joint parts can be held together securely after they are joined without any impairment of their rotational mobility.

According to this invention, bearing bushes made of plastic may be provided between the rotating parts to reduce friction.

In order to permit a secure connection between the bone and the shaft over a large area, the shafts may have an open-cell or open-pore surface structure into which the adjacent bone tissue of the bone cavity can grow in a preferred embodiment according to this invention.

In order to facilitate insertion of the axle into the boreholes of the hinge, the axle may be provided with a recess at one end for attaching a surgical tool such as forceps.

Appropriate devices such as stops may be provided on one of the two parts to work together with corresponding devices on the other part in order to limit the hinge movement.

This invention will be described in greater detail below with reference to the embodiments illustrated in the figures which show the following:
Figure 1 shows a sectional view of a middle interphalangeal (finger joint) prosthesis.
Figure 2 shows a sectional view of a middle interphalangeal prosthesis with a conical snap connector.
Figure 3 shows a side view of a middle interphalangeal joint prosthesis.
Figure 4 shows a side view of another embodiment of the middle interphalangeal joint prosthesis.
Figure 5 shows a front view of the middle interphalangeal joint prosthesis according to Figure 4 with a tapered first part.

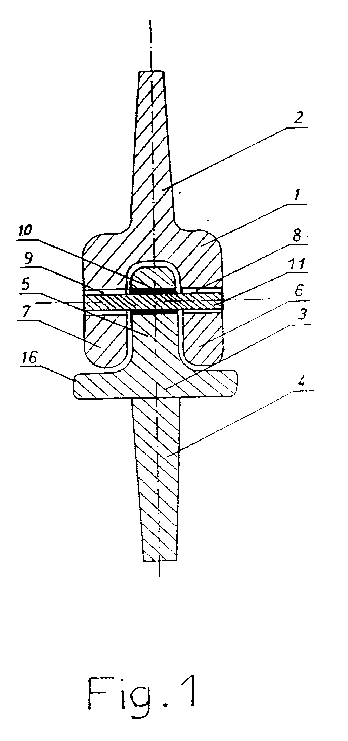

The middle interphalangeal joint prosthesis according to Figure 1 consists of a first part 1 and a second part 3. The two parts have shafts 2 and 4 on the opposite free ends that are inserted into the tubular bones of the corresponding finger bones and anchored there. Shafts 2 and 4 are designed with an open-pore and open-cell surface.

Part 1 is designed with two projections 6 and 7 with a distance between them on the fork end opposite shaft 2. Projections 6 and 7 are each provided with a borehole 8 or 9 of the same diameter at right angles to the longitudinal axis of the shaft 2.

Part 3 of the joint prosthesis has a web 5 that is aligned with shaft 4 on the end that is opposite shaft 4 and this web 5 is also provided with a borehole 10 of the same diameter as boreholes 8 and 9 aligned normal to the longitudinal axis of shaft 4.

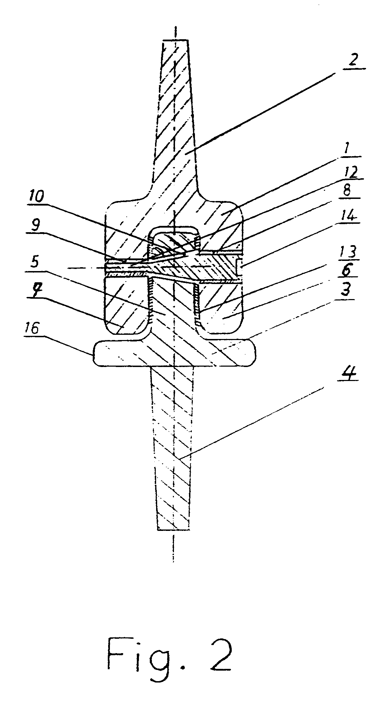

After inserting the shafts 2 and 4 into the tubular bones of the finger bones, the web 5 of part 3 is inserted into the interspace between the two projections 6 and 7 until the boreholes 8, 9 and 10 that are provided in all the parts are aligned. It is advantageous if the outer shape of the web 5 matches the shape of the interspace between the fork-shaped projections 6 and 7 in such a way that there is only a small play between the parts. A flat shape of web 5, which is inserted into the matching interspace of the first fork-shaped part, is especially advantageous, because the external dimensions of the finger joint are limited according to the anatomical requirements. In order to reduce friction between the rotating parts, plastic bushings 13 may also be inserted between them as shown in Figure 2.

Part 3 has a plate-shaped projection 16 across the axis of the shaft between projection 5 and shaft 4.

To form the hinge 30, an axle 11 is inserted through the boreholes 8, 9 and 10 in projections 5, 6 and 7, where the diameter of the axle corresponds approximately to the diameter of the boreholes 8, 9 and 10, whereby a slight play is allowed in one of the parts 1, 3, while the axle is securely anchored in the other part by means of a press fit, for example. Here again, it is possible to arrange plastic bearing bushes 13 between the parts between which there is a relative movement when there is a pivoting movement of the joint.

As an alternative to the embodiment in Figure 1 with a cylindrical axle, Figure 2 shows an axle 12 that is conical over a section of the length for joining hinge parts 1 and 3 and thus for forming the hinge 30. As shown in Figure 2, the middle longitudinal section of the axle 12 is designed with a conical taper, and after the parts 1 and 3 have been joined, the middle longitudinal section of axle 12 is in the borehole 10 of web 5, which has a matching conical shape. Consequently, boreholes 8 and 9 of projections 6 and 7 have different diameters, where the diameter of borehole 9 corresponds to the smaller diameter of the cone, and the diameter of borehole 8 corresponds to the larger diameter of the cone.

The conical borehole 10 and the corresponding conical section of axle 12 form a conical snap connector which assures that axle 12 is anchored securely in the joint after the hinge parts have been assembled.

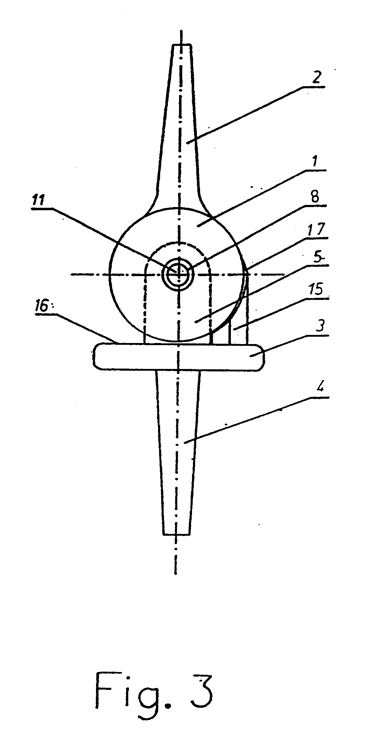

Figure 3 shows a side view of the joint prosthesis according to this invention. Stops 15 that work together with nose-shaped projections 17 on part 1 are provided on the plate-shaped part 16 on the side facing the back of the hand in order to limit the pivoting motion of the prosthetic middle joint in the desired manner.

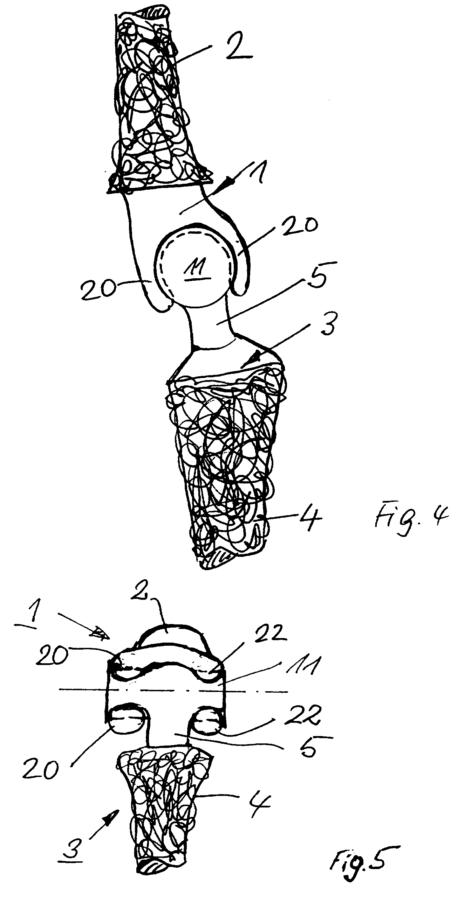

Figures 4 and 5 show another embodiment of the middle interphalangeal joint prosthesis according to this invention. On its free end the first part 1 has two claws 20, 22 that have a circular inside contour but are open on the free end over a given peripheral angle that is smaller than 180°.

The second part 3 of the prosthesis has a web 5 that projects between the claws 20, 22 which have some distance between them and has on its free end a transverse axle 11 that extends laterally into the claws 20, 22, where the transverse axle 11 has a slightly reduced diameter in the area of claws 20, 22 that corresponds to the inside diameter of claws 20, 22. The axial sections with a reduced inside diameter form peripheral grooves 11a, 11b in which claws 20, 22 engage with a snap action, and thus the second part 3 is held in place in such a way that it cannot slip axially. This design of claws 20, 22 and transverse axle 11 with radial grooves 11a, 11b yields a hinge joint whose two parts 1, 3 are hinge-connected together by means of a snap connector between claws 20, 22 and transverse axle.

## Claims

1. Prosthesis for replacing a middle interphalangeal joint, consisting of two parts joined together by means of a hinge, where each part has a shaft for anchoring in the adjacent finger bone, characterized in that the first part (1) and the second part (3) are joined together by means of a hinge (30).

2. Prosthesis according to Claim 1, characterized in that the first part (1) is designed as a fork having two projections (6, 7) with a distance between them and with aligned boreholes (8, 9), the second part (3) has a web (5) that is also provided with a borehole (10) and said web can be inserted into the interspace between projections (6, 7) on the first part (1), and an axle (12) is inserted into the aligned boreholes (8, 9 and 10) in order to form the hinge.

3. Prosthesis according to Claim 2, characterized in that the two projections (6, 7) of the first part (1) run parallel to each other.

4. Prosthesis according to one of the preceding claims, characterized in that the first part (1) and the second part (3) are hinge-connected to each other by means of an axle (12) that has a conical taper over the entire length or over just a portion of the longitudinal extent of the axle.

5. Prosthesis according to one of the preceding claims, characterized in that the borehole (8 or 9) in one of the fork-shaped projections (6 or 7) which receives the axle (12) said axle having a conical taper over a portion of the longitudinal extent itself has a conical taper.

6. Prosthesis according to one of the preceding claims, characterized in that the borehole (10) in the projection (5) of the second part (3) which receives the axle (12) said axle having a conical taper over a portion of its longitudinal extent is also designed with a conical taper.

7. Prosthesis according to one of the preceding claims, characterized in that all the boreholes (8, 9 and 10) which receive the axle (12) having a conical taper over its entire length are also designed with a conical taper.

8. Prosthesis according to one of the preceding claims, characterized in that bearing bushes (13) made of plastic are provided between the rotating parts.

9. Prosthesis according to Claim 1, characterized in that the surfaces of the shafts (2, 4) have an open-pore or open-cell surface structure.

10. Prosthesis according to Claim 1, characterized in that the attachment (5) on the second part (3) projects like a plate toward the shaft (4).

11. Prosthesis according to one or more of the preceding claims, characterized in that the axle (11 or 12) has a recess (14) on the end for attaching a surgical tool.

12. Prosthesis according to Claim 1, characterized in that one of the parts (1, 3) has devices for limiting the movement of the hinge.

13. Prosthesis according to Claim 1, characterized in that the axle (11) sits on the web (5), the first part (1) is designed as a fork with two claws (20, 22) on the end to reach around at least part of the axle (11).

14. Prosthesis according to Claim 13, characterized in that the axle (11) has radial grooves (11a, 11b) spaced the same distance apart as the claws (20, 22) so the claws (20, 22) together form a snap connection with said grooves.
